# EUROPEAN PATENT SPECIFICATION

(11) **EP 2 781 913 B1**
(45) Date of publication and mention of the grant of the patent: **13.12.2017**
(21) Application number: 14161288.7
(22) Date of filing: 11.09.2008
(51) Int. Cl.: G01N 27/00, B01D 59/44, G01N 33/68, G01N 33/574

(54) **Method for aiding in the diagnosis and therapy of asthma and lung cancer**
Verfahren zur Unterstützung der Diagnose und Therapy von Asthma und Lungenkrebs
Procédé pour aider le diagnostic et la thérapie de l'asthme et du cancer du poumon

(30) Priority: 11.09.2007 US 971422 P
(43) Date of publication of application: 24.09.2014
(62) Divisional of application: 08830503.2
(73) Proprietor: Cancer Prevention And Cure, Ltd., Michigan City, IN 46360 (US)
(72) Inventor: Streeper, Robert T., San Antonio, TX 78240 (US); Izbicka, Elzbieta, San Antonio, TX 78240 (US); Baek, Sung H., Snohomish, WA 98296 (US)
(74) Representative: Teall, Charlotte

(56) References cited:
- WO-A2-01/60857
- WO-A2-2004/035092
- DAI SONGWEI ET AL: "Discovery and identification of Serum Amyloid A protein elevated in lung cancer serum", SCIENCE IN CHINA SERIES C LIFE SCIENCES, vol. 50, no. 3, June 2007 (2007-06), pages 305-311, XP008134627, ISSN: 1006-9305

<110> Streeper, Robert T.; Izbicka, Elzbieta; Baek, Sung H.
<120> Method of Identifying Proteins in Human Serum Indicative of
   Pathologies of Human Lung Tissues
<130> P9286.2
<140>
   <141>
<150> US 60/971,422
   <151> 2007-09-11
<160> 11
<210> 1
   <211> 319
   <212> PRT
   <213> Homo sapien
<400> 1
   Met Val Leu Ser Glu Leu Ala Ala Arg Leu Asn Cys Ala Glu Tyr
                   5                   10                  15
   Lys Asn Trp Val Lys Ala Gly His Cys Leu Leu Leu Leu Arg Ser
                   20                  25                  30
   Cys Leu Gln Gly Phe Val Gly Arg Glu Val Leu Ser Phe His Arg
                   35                  40                  45
   Gly Leu Leu Ala Ala Ala Pro Gly Leu Gly Pro Arg Ala Val Cys
                   50                  55                  60
   Arg Gly Gly Ser Arg Cys Ser Pro Arg Ala Arg Gln Phe Gln Pro
                   65                  70                  75
   Gln Cys Gln Val Cys Ala Glu Trp Lys Arg Glu Ile Leu Arg His
                   80                  85                  90
   His Val Asn Arg Asn Gly Asp Val His Trp Gly Asn Cys Arg Pro
                   95                  100                 105
   Gly Arg Trp Pro Val Asp Ala Trp Glu Val Ala Lys Ala Phe Met
                   110                 115                 120
   Pro Arg Gly Leu Ala Asp Lys Gln Gly Pro Glu Glu Cys Asp Ala
                   125                 130                 135
   Val Ala Leu Leu Ser Leu Ile Asn Ser Cys Asp His Phe Val Val
                   140                 145                 150
   Asp Arg Lys Lys Val Thr Glu Val Ile Lys Cys Arg Asn Glu Ile
                   155                 160                 165
   Met His Ser Ser Glu Met Lys Val Ser Ser Thr Trp Leu Arg Asp
                   170                 175                 180
   Phe Gln Met Lys Ile Gln Asn Phe Leu Asn Glu Phe Lys Asn Ile
                   185                 190                 195
   Pro Glu Ile Val Ala Val Tyr Ser Arg Ile Glu Gln Leu Leu Thr
                   200                 205                 210
   Ser Asp Trp Ala Val His Ile Pro Glu Glu Asp Gln Arg Asp Gly
                   215                 220                 225
   Cys Glu Cys Glu Met Gly Thr Tyr Leu Ser Glu Ser Gln Val Asn
                   230                 235                 240
   Glu Ile Glu Met Gln Leu Leu Lys Glu Lys Leu Gln Glu Ile Tyr
                   245                 250                 255
   Leu Gln Ala Glu Glu Gln Glu Val Leu Pro Glu Glu Leu Ser Asn
                   260                 265                 270
   Arg Leu Glu Val Val Lys Glu Phe Leu Arg Asn Asn Glu Asp Leu
                   275                 280                 285
   Arg Asn Gly Leu Thr Glu Asp Met Gln Lys Leu Asp Ser Leu Cys
                   290                 295                 300
   Leu His Gln Lys Leu Asp Ser Gln Glu Pro Gly Arg Gln Thr Pro
                   305                 310                 315
   Asp Arg Lys Ala
<210> 2
   <211> 57
   <212> PRT
   <213> Homo sapiens
<400> 2
<210> 3
   <211> 62
   <212> PRT
   <213> Homo sapiens
<400> 3
   Met Ile Arg Ser Lys Phe Arg Val Pro Arg Ile Leu His Val Leu
                   5                   10                  15
   Ser Ala His Ser Gln Ala Ser Asp Lys Asn Phe Thr Ala Glu Asn
                   20                  25                  30
   Ser Glu Val Val Val Ser Ser Arg Thr Asp Val Ser Pro Met Lys
                   35                  40                  45
   Ser Asp Leu Leu Leu Pro Pro Ser Lys Pro Gly Cys Asn Asn Val
                   50                  55                  60
   Leu Asn
<210> 4
   <211> 146
   <212> PRT
   <213> Homo sapiens
<400> 4
   Met Val Gln Gly Met Cys Ser Pro Ser Pro Phe Gly Thr Ser Arg
                   5                   10                  15
   Ala Cys Thr Val Gly Thr Gln Val Asp Ser Arg Ser Leu Pro Trp
                   20                  25                  30
   Ala Leu Gly Ala Ser Ala Gln Arg Gly Asn Ile Pro Thr Ala Thr
                   35                  40                  45
   Cys Ala Arg Thr Ala Gly Thr Leu Arg Arg Gly Leu Gln Pro Gly
                   50                  55                  60
   Trp Gly Trp Glu Asp Phe Leu Asp Glu Gly Gln Pro Gly Phe Ser
                   65                  70                  75
   Ser Arg Met Ser Trp Ser Arg Pro Pro Ala Gln Glu Gln Gly Ala
                   80                  85                  90
   Gly Arg Gly Pro Ser Trp Val Arg Gly Leu Gly Gln Pro Thr Ala
                   95                  100                 105
   Ala Phe Glu Gln Gly Pro Arg Ser Ser Val Ser Pro Gln Trp Glu
                   110                 115                 120
   Gly Gly Gly Gln Gly Pro Gly Glu Leu Gly Arg Lys His Leu Leu
                   125                 130                 135
   Gly Pro Ser Gln His His Pro Thr Asp Arg His
                   140                 145
<210> 5
   <211> 1039
   <212> PRT
   <213> Homo sapiens
<400> 5
   Met Ala Val Gln Leu Val Pro Asp Ser Ala Leu Gly Leu Leu Met
                   5                   10                  15
   Met Thr Glu Gly Arg Arg Cys Gln Val His Leu Leu Asp Asp Arg
                   20                  25                  30
   Lys Leu Glu Leu Leu Val Gln Pro Lys Leu Leu Ala Lys Glu Leu
                   35                  40                  45
   Leu Asp Leu Val Ala Ser His Phe Asn Leu Lys Glu Lys Glu Tyr
                   50                  55                  60
   Phe Gly Ile Ala Phe Thr Asp Glu Thr Gly His Leu Asn Trp Leu
                   65                  70                  75
   Gln Leu Asp Arg Arg Val Leu Glu His Asp Phe Pro Lys Lys Ser
                   80                  85                  90
   Gly Pro Val Val Leu Tyr Phe Cys Val Arg Phe Tyr Ile Glu Ser
                   95                  100                 105
   Ile Ser Tyr Leu Lys Asp Asn Ala Thr Ile Glu Leu Phe Phe Leu
                   110                 115                 120
   Asn Ala Lys Ser Cys Ile Tyr Lys Glu Leu Ile Asp Val Asp Ser
                   125                 130                 135
   Glu Val Val Phe Glu Leu Ala Ser Tyr Ile Leu Gln Glu Ala Lys
                   140                 145                 150
   Gly Asp Phe Ser Ser Asn Glu Val Val Arg Ser Asp Leu Lys Lys
                   155                 160                 165
   Leu Pro Ala Leu Pro Thr Gln Ala Leu Lys Glu His Pro Ser Leu
                   170                 175                 180
   Ala Tyr Cys Glu Asp Arg Val Ile Glu His Tyr Lys Lys Leu Asn
                   185                 190                 195
   Gly Gln Thr Arg Gly Gln Ala Ile Val Asn Tyr Met Ser Ile Val
                   200                 205                 210
   Glu Ser Leu Pro Thr Tyr Gly Val His Tyr Tyr Ala Val Lys Asp
                   215                 220                 225
   Lys Gln Gly Ile Pro Trp Trp Leu Gly Leu Ser Tyr Lys Gly Ile
                   230                 235                 240
   Phe Gln Tyr Asp Tyr His Asp Lys Val Lys Pro Arg Lys Ile Phe
                   245                 250                 255
   Gln Trp Arg Gln Leu Glu Asn Leu Tyr Phe Arg Glu Lys Lys Phe
                   260                 265                 270
   Ser Val Glu Val His Asp Pro Arg Arg Ala Ser Val Thr Arg Arg
                   275                 280                 285
   Thr Phe Gly His Ser Gly Ile Ala Val His Thr Trp Tyr Ala Cys
                   290                 295                 300
   Pro Ala Leu Ile Lys Ser Ile Trp Ala Met Ala Ile Ser Gln His
                   305                 310                 315
   Gln Phe Tyr Leu Asp Arg Lys Gln Ser Lys Ser Lys Ile His Ala
                   320                 325                 330
   Ala Arg Ser Leu Ser Glu Ile Ala Ile Asp Leu Thr Glu Thr Gly
                   335                 340                 345
   Thr Leu Lys Thr Ser Lys Leu Ala Asn Met Gly Ser Lys Gly Lys
                   350                 355                 360
   Ile Ile Ser Gly Ser Ser Gly Ser Leu Leu Ser Ser Gly Ser Gln
                   365                 370                 375
   Glu Ser Asp Ser Ser Gln Ser Ala Lys Lys Asp Met Leu Ala Ala
                   380                 385                 390
   Leu Lys Ser Arg Gln Glu Ala Leu Glu Glu Thr Leu Arg Gln Arg
                   395                 400                 405
   Leu Glu Glu Leu Lys Lys Leu Cys Leu Arg Glu Ala Glu Leu Thr
                   410                 415                 420
   Gly Lys Leu Pro Val Glu Tyr Pro Leu Asp Pro Gly Glu Glu Pro
                   425                 430                 435
   Pro Ile Val Arg Arg Arg Ile Gly Thr Ala Phe Lys Leu Asp Glu
                   440                 445                 450
   Gln Lys Ile Leu Pro Lys Gly Glu Glu Ala Glu Leu Glu Arg Leu
                   455                 460                 465
   Glu Arg Glu Phe Ala Ile Gln Ser Gln Ile Thr Glu Ala Ala Arg
                   470                 475                 480
   Arg Leu Ala Ser Asp Pro Asn Val Ser Lys Lys Leu Lys Lys Gln
                   485                 490                 495
   Arg Lys Thr Ser Tyr Leu Asn Ala Leu Lys Lys Leu Gln Glu Ile
                   500                 505                 510
   Glu Asn Ala Ile Asn Glu Asn Arg Ile Lys Ser Gly Lys Lys Pro
                   515                 520                 525
   Thr Gln Arg Ala Ser Leu Ile Ile Asp Asp Gly Asn Ile Ala Ser
                   530                 535                 540
   Glu Asp Ser Ser Leu Ser Asp Ala Leu Val Leu Glu Asp Glu Asp
                   545                 550                 555
   Ser Gln Val Thr Ser Thr Ile Ser Pro Leu His Ser Pro His Lys
                   560                 565                 570
   Gly Leu Pro Pro Arg Pro Pro Ser His Asn Arg Pro Pro Pro Pro
                   575                 580                 585
   Gln Ser Leu Glu Gly Leu Arg Gln Met His Tyr His Arg Asn Asp
                   590                 595                 600
   Tyr Asp Lys Ser Pro Ile Lys Pro Lys Met Trp Ser Glu Ser Ser
                   605                 610                 615
   Leu Asp Glu Pro Tyr Glu Lys Val Lys Lys Arg Ser Ser His Ser
                   620                 625                 630
   His Ser Ser Ser His Lys Arg Phe Pro Ser Thr Gly Ser Cys Ala
                   635                 640                 645
   Glu Ala Gly Gly Gly Ser Asn Ser Leu Gln Asn Ser Pro Ile Arg
                   650                 655                 660
   Gly Leu Pro His Trp Asn Ser Gln Ser Ser Met Pro Ser Thr Pro
                   665                 670                 675
   Asp Leu Arg Val Arg Ser Pro His Tyr Val His Ser Thr Arg Ser
                   680                 685                 690
   Val Asp Ile Ser Pro Thr Arg Leu His Ser Leu Ala Leu His Phe
                   695                 700                 705
   Arg His Arg Ser Ser Ser Leu Glu Ser Gln Gly Lys Leu Leu Gly
                   710                 715                 720
   Ser Glu Asn Asp Thr Gly Ser Pro Asp Phe Tyr Thr Pro Arg Thr
                   725                 730                 735
   Arg Ser Ser Asn Gly Ser Asp Pro Met Asp Asp Cys Ser Ser Cys
                   740                 745                 750
   Thr Ser His Ser Ser Ser Glu His Tyr Tyr Pro Ala Gln Met Asn
                   755                 760                 765
   Ala Asn Tyr Ser Thr Leu Ala Glu Asp Ser Pro Ser Lys Ala Arg
                   770                 775                 780
   Gln Arg Gln Arg Gln Arg Gln Arg Ala Ala Gly Ala Leu Gly Ser
                   785                 790                 795
   Ala Ser Ser Gly Ser Met Pro Asn Leu Ala Ala Arg Gly Gly Ala
                   800                 805                 810
   Gly Gly Ala Gly Gly Ala Gly Gly Gly Val Tyr Leu His Ser Gln
                   815                 820                 825
   Ser Gln Pro Ser Ser Gln Tyr Arg Ile Lys Glu Tyr Pro Leu Tyr
                   830                 835                 840
   Ile Glu Gly Gly Ala Thr Pro Val Val Val Arg Ser Leu Glu Ser
                   845                 850                 855
   Asp Gln Glu Gly His Tyr Ser Val Lys Ala Gln Phe Lys Thr Ser
                   860                 865                 870
   Asn Ser Tyr Thr Ala Gly Gly Leu Phe Lys Glu Ser Trp Arg Gly
                   875                 880                 885
   Gly Gly Gly Asp Glu Gly Asp Thr Gly Arg Leu Thr Pro Ser Arg
                   890                 895                 900
   Ser Gln Ile Leu Arg Thr Pro Ser Leu Gly Arg Glu Gly Ala His
                   905                 910                 915
   Asp Lys Gly Ala Gly Arg Ala Ala Val Ser Asp Glu Leu Arg Gln
                   920                 925                 930
   Trp Tyr Gln Arg Ser Thr Ala Ser His Lys Glu His Ser Arg Leu
                   935                 940                 945
   Ser His Thr Ser Ser Thr Ser Ser Asp Ser Gly Ser Gln Tyr Ser
                   950                 955                 960
   Thr Ser Ser Gln Ser Thr Phe Val Ala His Ser Arg Val Thr Arg
                   965                 970                 975
   Met Pro Gln Met Cys Lys Ala Thr Ser Ala Ala Leu Pro Gln Ser
                   980                 985                 990
   Gln Arg Ser Ser Thr Pro Ser Ser Glu Ile Gly Ala Thr Pro Pro
                   995                 1000                1005
   Ser Ser Pro His His Ile Leu Thr Trp Gln Thr Gly Glu Ala Thr
                   1010                1015                1020
   Glu Asn Ser Pro Ile Leu Asp Gly Ser Glu Ser Pro Pro His Gln
                   1025                1030                1035
   Ser Thr Asp Glu
<210> 6
   <211> 244
   <212> PRT
   <213> Homo sapiens
<400> 6
   Met Ala Ala Ala Ala Ser Pro Ala Ile Leu Pro Arg Leu Ala Ile
                   5                   10                  15
   Leu Pro Tyr Leu Leu Phe Asp Trp Ser Gly Thr Gly Arg Ala Asp
                   20                  25                  30
   Ala His Ser Leu Trp Tyr Asn Phe Thr Ile Ile His Leu Pro Arg
                   35                  40                  45
   His Gly Gln Gln Trp Cys Glu Val Gln Ser Gln Val Asp Gln Lys
                   50                  55                  60
   Asn Phe Leu Ser Tyr Asp Cys Gly Ser Asp Lys Val Leu Ser Met
                   65                  70                  75
   Gly His Leu Glu Glu Gln Leu Tyr Ala Thr Asp Ala Trp Gly Lys
                   80                  85                  90
   Gln Leu Glu Met Leu Arg Glu Val Gly Gln Arg Leu Arg Leu Glu
                   95                  100                 105
   Leu Ala Asp Thr Glu Leu Glu Asp Phe Thr Pro Ser Gly Pro Leu
                   110                 115                 120
   Thr Leu Gln Val Arg Met Ser Cys Glu Cys Glu Ala Asp Gly Tyr
                   125                 130                 135
   Ile Arg Gly Ser Trp Gln Phe Ser Phe Asp Gly Arg Lys Phe Leu
                   140                 145                 150
   Leu Phe Asp Ser Asn Asn Arg Lys Trp Thr Val Val His Ala Gly
                   155                 160                 165
   Ala Arg Arg Met Lys Glu Lys Trp Glu Lys Asp Ser Gly Leu Thr
                   170                 175                 180
   Thr Phe Phe Lys Met Val Ser Met Arg Asp Cys Lys Ser Trp Leu
                   185                 190                 195
   Arg Asp Phe Leu Met His Arg Lys Lys Arg Leu Glu Pro Thr Ala
                   200                 205                 210
   Pro Pro Thr Met Ala Pro Gly Leu Ala Gln Pro Lys Ala Ile Ala
                   215                 220                 225
   Thr Thr Leu Ser Pro Trp Ser Phe Leu Ile Ile Leu Cys Phe Ile
                   230                 235                 240
   Leu Pro Gly Ile
<210> 7
   <211> 536
   <212> PRT
   <213> Homo sapiens
<400> 7
   Met Glu Ile Arg Gln His Glu Trp Leu Ser Ala Ser Pro His Glu
                   5                   10                  15
   Gly Phe Glu Gln Met Arg Leu Lys Ser Arg Pro Lys Glu Pro Ser
                   20                  25                  30
   Pro Ser Leu Thr Arg Val Gly Ala Asn Phe Tyr Ser Ser Val Lys
                   35                  40                  45
   Gln Gln Asp Tyr Ser Ala Ser Val Trp Leu Arg Arg Lys Asp Lys
                   50                  55                  60
   Leu Glu His Ser Gln Gln Lys Cys Ile Val Ile Phe Ala Leu Val
                   65                  70                  75
   Cys Cys Phe Ala Ile Leu Val Ala Leu Ile Phe Ser Ala Val Asp
                   80                  85                  90
   Ile Met Gly Glu Asp Glu Asp Gly Leu Ser Glu Lys Asn Cys Gln
                   95                  100                 105
   Asn Lys Cys Arg Ile Ala Leu Val Glu Asn Ile Pro Glu Gly Leu
                   110                 115                 120
   Asn Tyr Ser Glu Asn Ala Pro Phe His Leu Ser Leu Phe Gln Gly
                   125                 130                 135
   Trp Met Asn Leu Leu Asn Met Ala Lys Lys Ser Val Asp Ile Val
                   140                 145                 150
   Ser Ser His Trp Asp Leu Asn His Thr His Pro Ser Ala Cys Gln
                   155                 160                 165
   Gly Gln Arg Leu Phe Glu Lys Leu Leu Gln Leu Thr Ser Gln Asn
                   170                 175                 180
   Ile Glu Ile Lys Leu Val Ser Asp Val Thr Ala Asp Ser Lys Val
                   185                 190                 195
   Leu Glu Ala Leu Lys Leu Lys Gly Ala Glu Val Thr Tyr Met Asn
                   200                 205                 210
   Met Thr Ala Tyr Asn Lys Gly Arg Leu Gln Ser Ser Phe Trp Ile
                   215                 220                 225
   Val Asp Lys Gln His Val Tyr Ile Gly Ser Ala Gly Leu Asp Trp
                   230                 235                 240
   Gln Ser Leu Gly Gln Met Lys Glu Leu Gly Val Ile Phe Tyr Asn
                   245                 250                 255
   Cys Ser Cys Leu Val Leu Asp Leu Gln Arg Ile Phe Ala Leu Tyr
                   260                 265                 270
   Ser Ser Leu Lys Phe Lys Ser Arg Val Pro Gln Thr Trp Ser Lys
                   275                 280                 285
   Arg Leu Tyr Gly Val Tyr Asp Asn Glu Lys Lys Leu Gln Leu Gln
                   290                 295                 300
   Leu Asn Glu Thr Lys Ser Gln Ala Phe Val Ser Asn Ser Pro Lys
                   305                 310                 315
   Leu Phe Cys Pro Lys Asn Arg Ser Phe Asp Ile Asp Ala Ile Tyr
                   320                 325                 330
   Ser Val Ile Asp Asp Ala Lys Gln Tyr Val Tyr Ile Ala Val Met
                   335                 340                 345
   Asp Tyr Leu Pro Ile Ser Ser Thr Ser Thr Lys Arg Thr Tyr Trp
                   350                 355                 360
   Pro Asp Leu Asp Ala Lys Ile Arg Glu Ala Leu Val Leu Arg Ser
                   365                 370                 375
   Val Arg Val Arg Leu Leu Leu Ser Phe Trp Lys Glu Thr Asp Pro
                   380                 385                 390
   Leu Thr Phe Asn Phe Ile Ser Ser Leu Lys Ala Ile Cys Thr Glu
                   395                 400                 405
   Ile Ala Asn Cys Ser Leu Lys Val Lys Phe Phe Asp Leu Glu Arg
                   410                 415                 420
   Glu Asn Ala Cys Ala Thr Lys Glu Gln Lys Asn His Thr Phe Pro
                   425                 430                 435
   Arg Leu Asn Arg Asn Lys Tyr Met Val Thr Asp Gly Ala Ala Tyr
                   440                 445                 450
   Ile Gly Asn Phe Asp Trp Val Gly Asn Asp Phe Thr Gln Asn Ala
                   455                 460                 465
   Gly Thr Gly Leu Val Ile Asn Gln Ala Asp Val Arg Asn Asn Arg
                   470                 475                 480
   Ser Ile Ile Lys Gln Leu Lys Asp Val Phe Glu Arg Asp Trp Tyr
                   485                 490                 495
   Ser Pro Tyr Ala Lys Thr Leu Gln Pro Thr Lys Gln Pro Asn Cys
                   500                 505                 510
   Ser Ser Leu Phe Lys Leu Lys Pro Leu Ser Asn Lys Thr Ala Thr
                   515                 520                 525
   Asp Asp Thr Gly Gly Lys Asp Pro Arg Asn Val
                   530                 535
<210> 8
   <211> 344
   <212> PRT
   <213> Homo sapiens
<400> 8
   Gln Asn Leu Pro Ser Ser Pro Ala Pro Ser Thr Ile Phe Ser Gly
                   5                   10                  15
   Gly Phe Arg His Gly Ser Leu Ile Ser Ile Asp Ser Thr Cys Thr
                   20                  25                  30
   Glu Met Gly Asn Phe Asp Asn Ala Asn Val Thr Gly Glu Ile Glu
                   35                  40                  45
   Phe Ala Ile His Tyr Cys Phe Lys Thr His Ser Leu Glu Ile Cys
                   50                  55                  60
   Ile Lys Ala Cys Lys Asn Leu Ala Tyr Gly Glu Glu Lys Lys Lys
                   65                  70                  75
   Lys Cys Asn Pro Tyr Val Lys Thr Tyr Leu Leu Pro Asp Arg Ser
                   80                  85                  90
   Ser Gln Gly Lys Arg Lys Thr Gly Val Gln Arg Asn Thr Val Asp
                   95                  100                 105
   Pro Thr Phe Gln Glu Thr Leu Lys Tyr Gln Val Ala Pro Ala Gln
                   110                 115                 120
   Leu Val Thr Arg Gln Leu Gln Val Ser Val Trp His Leu Gly Thr
                   125                 130                 135
   Leu Ala Arg Arg Val Phe Leu Gly Glu Val Ile Ile Ser Leu Ala
                   140                 145                 150
   Thr Trp Asp Phe Glu Asp Ser Thr Thr Gln Ser Phe Arg Trp His
                   155                 160                 165
   Pro Leu Arg Ala Lys Ala Glu Lys Tyr Glu Asp Ser Val Pro Gln
                   170                 175                 180
   Ser Asn Gly Glu Leu Thr Val Arg Ala Lys Leu Val Leu Pro Ser
                   185                 190                 195
   Arg Pro Arg Lys Leu Gln Glu Ala Gln Glu Gly Thr Asp Gln Pro
                   200                 205                 210
   Ser Leu His Gly Gln Leu Cys Leu Val Val Leu Gly Ala Lys Asn
                   215                 220                 225
   Leu Pro Val Arg Pro Asp Gly Thr Leu Asn Ser Phe Val Lys Gly
                   230                 235                 240
   Cys Leu Thr Leu Pro Asp Gln Gln Lys Leu Arg Leu Lys Ser Pro
                   245                 250                 255
   Val Leu Arg Lys Gln Ala Cys Pro Gln Trp Lys His Ser Phe Val
                   260                 265                 270
   Phe Ser Gly Val Thr Pro Ala Gln Leu Arg Gln Ser Ser Leu Glu
                   275                 280                 285
   Leu Thr Val Trp Asp Gln Ala Leu Phe Gly Met Asn Asp Arg Leu
                   290                 295                 300
   Leu Gly Gly Thr Arg Leu Gly Ser Lys Gly Asp Thr Ala Val Gly
                   305                 310                 315
   Gly Asp Ala Cys Ser Leu Ser Lys Leu Gln Trp Gln Lys Val Leu
                   320                 325                 330
   Ser Ser Pro Asn Leu Trp Thr Asp Met Thr Leu Val Leu His
<210> 9
   <211> 263
   <212> PRT
   <213> Homo sapiens
<400> 9
   Met Phe Arg Asn Gln Tyr Asp Asn Asp Val Thr Val Trp Ser Pro
                   5                   10                  15
   Gln Gly Arg Ile His Gln Ile Glu Tyr Ala Met Glu Ala Val Lys
                   20                  25                  30
   Gln Gly Ser Ala Thr Val Gly Leu Lys Ser Lys Thr His Ala Val
                   35                  40                  45
   Leu Val Ala Leu Lys Arg Ala Gln Ser Glu Leu Ala Ala His Gln
                   50                  55                  60
   Lys Lys Ile Leu His Val Asp Asn His Ile Gly Ile Ser Ile Ala
                   65                  70                  75
   Gly Leu Thr Ala Asp Ala Arg Leu Leu Cys Asn Phe Met Arg Gln
                   80                  85                  90
   Glu Cys Leu Asp Ser Arg Phe Val Phe Asp Arg Pro Leu Pro Val
                   95                  100                 105
   Ser Arg Leu Val Ser Leu Ile Gly Ser Lys Thr Gln Ile Pro Thr
                   110                 115                 120
   Gln Arg Tyr Gly Arg Arg Pro Tyr Gly Val Gly Leu Leu Ile Ala
                   125                 130                 135
   Gly Tyr Asp Asp Met Gly Pro His Ile Phe Gln Thr Cys Pro Ser
                   140                 145                 150
   Ala Asn Tyr Phe Asp Cys Arg Ala Met Ser Ile Gly Ala Arg Ser
                   155                 160                 165
   Gln Ser Ala Arg Thr Tyr Leu Glu Arg His Met Ser Glu Phe Met
                   170                 175                 180
   Glu Cys Asn Leu Asn Glu Leu Val Lys His Gly Leu Arg Ala Leu
                   185                 190                 195
   Arg Glu Thr Leu Pro Ala Glu Gln Asp Leu Thr Thr Lys Asn Val
                   200                 205                 210
   Ser Ile Gly Ile Val Gly Lys Asp Leu Glu Phe Thr Ile Tyr Asp
                   215                 220                 225
   Asp Asp Asp Val Ser Pro Phe Leu Glu Gly Leu Glu Glu Arg Pro
                   230                 235                 240
   Gln Arg Lys Ala Gln Pro Ala Gln Pro Ala Asp Glu Pro Ala Glu
                   245                 250                 255
   Lys Ala Asp Glu Pro Met Glu His
                   260
<210> 10
   <211> 287
   <212> PRT
   <213> Homo sapiens
<400> 10
<210> 11
   <211> 244
   <212> PRT
   <213> Homo sapiens
<400> 11
   Met Ala Ala Ala Ala Ser Pro Ala Ile Leu Pro Arg Leu Ala Ile
   5 10 15
   Leu Pro Tyr Leu Leu Phe Asp Trp Ser Gly Thr Gly Arg Ala Asp
   20 25 30
   Ala His Ser Leu Trp Tyr Asn Phe Thr Ile Ile His Leu Pro Arg
   35 40 45
   His Gly Gln Gln Trp Cys Glu Val Gln Ser Gln Val Asp Gln Lys
   50 55 60
   Asn Phe Leu Ser Tyr Asp Cys Gly Ser Asp Lys Val Leu Ser Met
                   65                  70                  75
   Gly His Leu Glu Glu Gln Leu Tyr Ala Thr Asp Ala Trp Gly Lys
                   80                  85                  90
   Gln Leu Glu Met Leu Arg Glu Val Gly Gln Arg Leu Arg Leu Glu
                   95                  100                 105
   Leu Ala Asp Thr Glu Leu Glu Asp Phe Thr Pro Ser Gly Pro Leu
                   110                 115                 120
   Thr Leu Gln Val Arg Met Ser Cys Glu Cys Glu Ala Asp Gly Tyr
                   125                 130                 135
   Ile Arg Gly Ser Trp Gln Phe Ser Phe Asp Gly Arg Lys Phe Leu
                   140                 145                 150
   Leu Phe Asp Ser Asn Asn Arg Lys Trp Thr Val Val His Ala Gly
                   155                 160                 165
   Ala Arg Arg Met Lys Glu Lys Trp Glu Lys Asp Ser Gly Leu Thr
                   170                 175                 180
   Thr Phe Phe Lys Met Val Ser Met Arg Asp Cys Lys Ser Trp Leu
                   185                 190                 195
   Arg Asp Phe Leu Met His Arg Lys Lys Arg Leu Glu Pro Thr Ala
                   200                 205                 210
   Pro Pro Thr Met Ala Pro Gly Leu Ala Gln Pro Lys Ala Ile Ala
                   215                 220                 225
   Thr Thr Leu Ser Pro Trp Ser Phe Leu Ile Ile Leu Cys Phe Ile
                   230                 235                 240
   Leu Pro Gly Ile

## Description

### BACKGROUND OF THE INVENTION

### 1. Field of the Invention

The present invention as defined in the claims relates generally to methods aiding in the diagnosis of pathologies of human lung tissues. More specifically, the present invention relates to methods aiding in the diagnosis of non-small cell lung cancers and asthma using liquid chromatography-mass spectrometry to identify proteins present in human sera which, when altered in terms of relative intensity of expression in the human serum from the same proteins found in a normal population, are indicative of pathologies associated with human lung tissues and the human respiratory system. By identifying the proteins associated with such pathologies, determining representative expression intensities, and comparing those expression intensities to the expression intensities present in the serum of a patient, it is possible to detect the presence of the pathologies early on in their progression through simple blood tests and to differentiate among the pathologies.

### 2. Description of the Related Art

Pathologies of the respiratory system, such as asthma and lung cancer, affect millions of Americans. In fact, the American Lung Association reports that almost 20 million Americans suffer from asthma. The American Cancer Society estimated 229,400 new cancer cases of the respiratory system and 164,840 deaths from cancers of the respiratory system in 2007 alone. While the five year survival rate of cancer cases when the cancer is detected while still localized is 46%, the five year survival rate of lung cancer patients is only 13%. Correspondingly, only 16% of lung cancers are discovered before the disease has spread. Lung cancers are generally categorised as two main types based on the pathology of the cancer cells. Each type is named for the types of cells that were transformed to become cancerous. Small cell lung cancers are derived from small cells in the human lung tissues, whereas non-small lung cancers generally encompass all lung cancers that are not small-cell type. Non-small cell lung cancers are grouped together because the treatment is generally the same for all non-small-cell types. Together, non-small-cell lung cancers, or NSCLCs, make up about 75% of all lung cancers.

A major factor in the diminishing survival rate of lung cancer patients is the fact that lung cancer is difficult to diagnose early. Current methods of diagnosing lung cancer or identifying its existence in a human are restricted to taking X-rays, CT scans and similar tests of the lungs to physically determine the presence or absence of a tumor. Therefore, the diagnosis of lung cancer is often made only in response to symptoms which have presented for a significant period of time, and after the disease has been present in the human long enough to produce a physically detectable mass.

Similarly, current methods of detecting asthma are typically performed long after the presentation of symptoms such as recurrent wheezing, coughing, and chest tightness. Current methods of detecting asthma are typically restricted to lung function tests such as spirometry tests or challenge tests. Moreover, these tests are often ordered by the physician to be performed along with the multitude of other tests to rule out other pathologies or diseases such as chronic obstructive pulmonary disease (COPD), bronchitis, pneumonia, and congestive heart failure.

There does not exist in the prior art a simple, reliable method of diagnosing pathologies of human lung tissues early in their development. Furthermore, there is not a blood test available today which is capable of indicating the presence of a particular lung tissue pathology. It is therefore to develop a method to determine the existence of lung cancers early in the disease progression. It is likewise desirable to develop a method to diagnose asthma and non-small cell lung cancer and to differentiate them from each other and from other lung diseases such as infections at the earliest appearance of symptoms. It is further desirable to identify specific proteins present in human blood which, when altered in terms of relative intensities of expression, are indicative of the presence of non-small cell lung cancers and/or asthma.

WO01/60857 is a document disclosing the same protein (here called PAP1) as described in SEQ ID NO:8 (see claim 8, abstract) but no link to lung disease is made here.

Dai Songwei et al. (06-2007), Science in China, Series C Life Sciences, vol. 50, no. 3, p.305-311 discloses markers for lung cancer and asthma but no link to the protein of SEQ ID NO:8 is made.

### Summary of the invention

The invention in its broadest form is defined by independent claims 1 and 3:

A method aiding in the diagnosis of asthma and/or lung cancer pathologies in patients early in the progression of the diseases comprising determining the specific protein concentrations of CAC69571 (SEQ ID NO: 8), and optionally one or more of the proteins FERM domain containing proteins 4 (SEQ ID NO: 5), JC1445 proteasome endopetidase complex chain C2 long splice (SEQ ID NO: 9), Syntaxin 11 (SEQ ID NO: 10), AAK13083 (SEQ ID NO: 6), AAK13049 (SEQ ID NO: 11), BACO4615 (SEQ ID NO: 1), Q6NSC8 (SEQ ID NO: 2), CAF17350 (SEQ ID NO: 3), Q6ZUD4 (SEQ ID NO: 4), and/or Q8N7P1 (SEQ ID NO: 7) in a patient's serum and comparing the concentration data to concentration data from lung cancer populations and/or asthma populations to verify or nullify the presence of the given pathologies.

A method to evaluate the response of a patient to therapeutic interventions comprising determining the specific protein concentrations of CAC69571 (SEQ ID NO: 8), and optionally one or more of the proteins FERM domain containing proteins 4 (SEQ ID NO: 5), JC1445 proteasome endopetidase complex chain C2 long splice (SEQ ID NO: 9), Syntaxin 11 (SEQ ID NO: 10), AAK13083 (SEQ ID NO: 6), AAK13049 (SEQ ID NO: 11), BACO4615 (SEQ ID NO: 1), Q6NSC8 (SEQ ID NO: 2), CAF17350 (SEQ ID NO: 3), Q6ZUD4 (SEQ ID NO: 4), and/or Q8N7P1 (SEQ ID NO: 7) in a patient's serum, wherein the patient is an asthma or lung cancer patient. Preferred embodiments are defined in dependent claims 2, 4 and 5.

### FURTHER ASPECTS OF THE CURRENT DISCLOSURE

The present specification provides a novel method of identifying proteins present in human serum which are differentially expressed between normal individuals and patients known to have non-small cell lung cancers and asthma, as diagnosed by a physician, using a liquid chromatography electrospray ionization mass spectrometer ("LC-ESIMS"). Selection of proteins indicative of non-small cell lung cancers and/or asthma was made by comparing the mass spectral data, namely the mass of peptides and graphical indications of the intensities of the proteins expressed across time in a single dimension. Thousands of proteins were compared, resulting in the selection of eleven proteins which were expressed in substantially differing intensities between populations of individuals not having any lung tissue pathologies, populations of individuals having asthma, as diagnosed by a physician, and populations of individuals having non-small cell lung cancers, as diagnosed by a physician.

Specifically, human sera were obtained from a "normal population," an "asthma population", and a "lung cancer population." "Normal population," as used herein is meant to define those individuals known not to have asthma or lung cancers. "Asthma population," as used herein, is meant to define those individuals which were known to have asthma and diagnosed as such by a physician. "Lung cancer population," as used herein, is meant to define those individuals which were known to have non-small cell lung cancers and diagnosed as such by a physician.

After obtaining the sera of the normal population, asthma population and lung cancer population, each serum specimen was divided into aliquots and exposed to a digesting agent or protease, namely, trypsin, to digest the proteins present in the serum specimens into defined and predictable cleavages or peptides. The peptides created by the enzymatic action of trypsin, commonly known as the tryptic peptides, were then separated from the insoluble matter digested by the trypsin by subjecting the specimens to a centrifugation to precipitate insoluble matter. The supernatant solution containing the tryptic peptides was then subjected to capillary liquid chromatography to effect tempero-spatial separation of the tryptic peptides.

The tryptic peptides were then subjected to an LC-ESIMS. Each peptide was separated in time by passing the peptide through a column of hydrophobic fluid, namely, water, acetonitrile containing 0.1% by volume formic acid over a chromatographic column containing Supelcosil ABZ+ 5 µm packing material stationary phase with a bed length of 18 cm and an internal diameter of 0.375 mm. The separated peptides are carried by a column effluent. The column has a terminus from which the separated peptides were then electrosprayed by application of a high voltage to the column tip having a positive bias relative to ground, forming a beam of charged droplets that were accelerated toward the inlet of the LC-ESIMS by the force of the applied electrical field. The resulting spray formed consisted of small droplets of solvent containing dissolved tryptic peptides. The droplets were desolvated by passage across an atmospheric pressure region of the electrospray source and then into a heated capillary inlet of the LC-ESIMS.

The desolvation of the droplets resulted in the deposition of positively charged ions, most typically hydrogen (H⁺) on the peptides, imparting charge to the peptides. Such charged peptides in the gas phase are described in the art as "pseudo-molecular ions." The pseudo-molecular ions are drawn through various electrical potentials into the mass analyzer of the LC-ESIMS, wherein they are separated in space and time on the basis of the mass to charge ratio. Once separated by mass to charge ratio, the pseudo-molecular ions are then directed by additional electric field gradients into a detector of the LC-ESIMS, wherein the pseudo-molecular ion beam is converted into electrical impulses that are recorded by data recording devices.

Thus, the peptides present in the tryptic digest were passed to the mass analyzer in the LC-ESIMS where molecular weights were measured for each peptide, producing time incremented mass spectra that are acquired repeatedly over the entirety of the time that the peptides from the sample are passing out of the column. The mass spectral readouts are generally graphic illustrations of the peptides found by the LC-ESIMS, wherein the x-axis is the measurement mass to charge ratio, the y-axis is the signal intensity of the peptide. These mass spectra can then be assembled in time into a three dimensional display wherein the x-axis is the time of the chromatographic separation, the z-axis is the mass axis of the mass spectrum and the y-axis is the intensity of the mass spectral signals, which is proportional to the quantity of a given pseudo-molecular ion detected by the LC-ESIMS.

Next, comparative analysis was performed comparing the mass spectral readouts for each specimen tested from the asthma population and the lung cancer population to each specimen tested from the normal population. Each tryptic peptide pseudo-molecular ion signal ("peak") associated with a putatively identified protein that was detected in the LC-ESIMS was compared across asthma, lung cancer and normal pathologies. Peptides with mass spectral peak intensities that indicated the peptide quantities were not substantially altered when comparing the asthma population or lung cancer population to the normal population were determined to be insignificant and excluded. Generally, the exclusion criteria used involved comparing the peptide peak intensities for at least half of the identified characteristic peptides for a given protein across at least ten data sets derived from the analysis of individual patient sera from each pathology. If the intensity of the majority of peptide peaks derived from given protein were at least 10 fold higher in intensity for 80% of the serum data sets, the protein was classed as differentially regulated between the two pathologic classes.

As a result of the comparative analysis, eleven proteins were determined to be consistently differentially expressed between the asthma population, lung cancer population and normal population. The eleven proteins were identified by reference to known databases or libraries of proteins and peptides. Examples of such databases include Entrez Protein maintained by the National Center for Biotechnology Information "NCBInr"), ExPASy maintained by the Swiss Bioinformatics Institute ("SwissProt"), and the Mass Spectral Database ("MSDB") of the Medical Research Council Clinical Science Center of the Imperial College of London.

The mass spectral readouts for each specimen from each of the normal, lung cancer and asthma population were inputted into a known search engine called Mascot. Mascot is a search engine known in the art which uses mass spectrometry data to identify proteins from four major sequencing databases, namely the MSDB, NCBInr, SwissProt and dbEST databases. Search criteria and parameters were inputted into the Mascot program and each specimen was run through the Mascot program. The Mascot program then ran the peptides inputted against the sequencing databases, comparing the peak intensities and masses of each peptide to the masses and peak intensities of known peptides and proteins. Mascot then produced a candidate list of possible matches, commonly known as "significant matches" for each peptide that was run.

Significant matches are determined by the Mascot program by assigning a score called a "Mowse score" for each specimen tested. The Mowse score is an algorithm wherein the score is -10*LOG₁₀(P), where P is the probability that the observed match is a random event, which correlates into a significance p value where p is less than 0.05, which is the generally accepted standard of significance in the scientific community. Mowse scores of approximately 55 to approximately 66 or greater are generally considered significant. The significance level varies somewhat due to specific search considerations and database parameters. The significant matches were returned for each peptide run, resulting in a candidate list of proteins.

The peptides were then matched to the proteins from the significant matches to determine the identity of the peptides run through the Mascot program. Manual analysis was performed for each peptide identified by the Mascot program and each protein from the significant matches. The peak intensity matches which were determined to be the result of "noise", whether chemical or electronic were excluded. The data from the mass spectral readouts were cross checked with the significant matches to confirm the raw data, peak identities, charge multiplicities, isotope distribution and flanking charge states.

A reverse search was then performed to add peptides to the candidate list which may have been missed by the automated search through the Mascot program. The additional peptides were identified by selecting the "best match" meaning the single protein which substantially matched each parameter of the peptide compared, performing an *in silico* digest wherein the tryptic peptides and their respective molecular masses are calculated based on the known amino acid or gene sequence of the protein. These predicted peptide masses are then searched against the raw mass spectral data and any peaks identified are examined and qualified as described above. Then, all of the peptides including those automatically identified by Mascot and those identified by manual examination are entered into the mass list used by Mascot. The refined match is then used to derive the refined Mowse score, as discussed herein below.

As a result of the identification process, the eleven proteins determined to be significantly differentially expressed between the asthma population, lung cancer population and/or normal population were identified as BAC04615, Q6NSC8, CAF17350, Q6ZUD4, Q8N7P1, CAC69571, FERM domain containing protein 4, JC1445 proteasome endopetidase complex chain C2 long splice, Syntaxin 11, AAK13083, and AAK130490. BAC04615, Q6NSC8, CAF 17350, Q6ZUD4, Q8N7P1 are identified proteins resulting from genetic sequencing efforts. FERM domain containing protein 4 is known to be involved in intracytoplasmic protein membrane anchorage. JC1445 proteasome endopetidase complex chain C2 long splice is a known proteasome. Syntaxin 11 is active in cellular immune response. BAC04615, AAK13083, and AAK130490 are major histocompatibility complex ("MHC") associated proteins.

### BRIEF DESCRIPTION OF THE DRAWINGS

Fig. 1 discloses a table showing Mowse scores and significant matches for the protein BAC04615;
Fig. 2 discloses a table showing Mowse scores and significant matches for the protein Q6NSC8;
Fig. 3 discloses a table showing Mowse scores and significant matches for the protein CAF17350;
Fig. 4 discloses a table showing Mowse scores and significant matches for the protein Q6ZUD4;
Fig. 5 discloses a table showing Mowse scores and significant matches for the protein Q8N7P1;
Fig. 6 discloses a table showing Mowse scores and significant matches for the protein CAC69571;
Fig. 7 discloses a table showing Mowse scores and significant matches for the protein FERM 4 domain containing protein 4;
Fig. 8 discloses a table showing Mowse scores and significant matches for the protein JC1445 proteasome endopetidase complex chain C2 long splice;
Fig. 9 discloses a table showing Mowse scores and significant matches for the protein Syntaxin 11.
Fig. 10 discloses a table showing Mowse scores and significant matches for the proteins AAK13083 and AAK13049.

### DETAILED DESCRIPTION OF THE INVENTION

The present invention in its broadest form is defined in independent claims 1 and 3:

A method aiding in the diagnosis of asthma and/or lung cancer pathologies in patients early in the progression of the diseases comprising determining the specific protein concentrations of CAC69571 (SEQ ID NO: 8), and optionally one or more of the proteins FERM domain containing proteins 4 (SEQ ID NO: 5), JC1445 proteasome endopetidase complex chain C2 long splice (SEQ ID NO: 9), Syntaxin 11 (SEQ ID NO: 10), AAK13083 (SEQ ID NO: 6), AAK13049 (SEQ ID NO: 11), BACO4615 (SEQ ID NO: 1), Q6NSC8 (SEQ ID NO: 2), CAF17350 (SEQ ID NO: 3), Q6ZUD4 (SEQ ID NO: 4), and/or Q8N7P1 (SEQ ID NO: 7) in a patient's serum and comparing the concentration data to concentration data from lung cancer populations and/or asthma populations to verify or nullify the presence of the given pathologies.

A method to evaluate the response of a patient to therapeutic interventions comprising determining the specific protein concentrations of CAC69571 (SEQ ID NO: 8), and optionally one or more of the proteins FERM domain containing proteins 4 (SEQ ID NO: 5), JC1445 proteasome endopetidase complex chain C2 long splice (SEQ ID NO: 9), Syntaxin 11 (SEQ ID NO: 10), AAK13083 (SEQ ID NO: 6), AAK13049 (SEQ ID NO: 11), BACO4615 (SEQ ID NO: 1), Q6NSC8 (SEQ ID NO: 2), CAF17350 (SEQ ID NO: 3), Q6ZUD4 (SEQ ID NO: 4), and/or Q8N7P1 (SEQ ID NO: 7) in a patient's serum, wherein the patient is an asthma or lung cancer patient.

Preferred embodiments are defined in dependent claims 2, 4 and 5.

The present invention provides a method of identifying, and identifies proteins present in human serum which are differentially expressed between normal individuals and patients known to have non-small cell lung cancers and asthma, as diagnosed by a physician, using liquid chromatography electrospray ionization mass spectrometry. By determining the proteins which are substantially and consistently differentially expressed between populations of people not having any pathologies of human lung tissues, populations of people diagnosed with asthma, and populations of people diagnosed with non-small cell lung cancers, and obtaining the identity of those proteins, it is possible to identify the presence of the pathology in a patient through blood tests identifying the same proteins and quantifying the expression levels of the proteins to identify and diagnose asthma or non-small cell lung cancer much earlier in the progression of the respective diseases.

Human blood samples were collected from volunteers. Thirty samples were collected from individuals known not to have either non-small cell lung cancer or asthma. The individuals known not to have either non-small cell lung cancer or asthma comprise, and are referred to herein as, the "normal population". Furthermore, the term "lung cancer", as used herein, is meant to describe non-small cell lung cancers. Twenty-eight blood samples were collected from individuals known to have asthma and diagnosed as such by a physician. The individuals known to have asthma comprise, and are referred to herein as, the "asthma population." Thirty blood samples were collected from individuals known to have non-small cell lung cancers and diagnosed as such by a physician. The individuals known to have non-small cell lung cancer comprise, and are referred to herein, as the "lung cancer population." Generally, as used herein, the term "lung cancer" or "lung cancers" is meant to refer to non-small cell lung cancers. Finally, seventy-one blood samples were collected from individuals known to have risks of lung cancer due to a history of cigarette smoking as recorded by a physician. These seventy one samples are the subject of ongoing research and experimentation, and are accordingly not discussed herein.

The blood samples were collected from volunteers under an IRB approved protocol, following informed consent using standard venipuncture techniques into sterile 10 ml BD Vacutainer® glass serum red top tubes. The blood samples were then left undisturbed at room temperature for thirty minutes to allow the blood to clot. The samples were spun in a standard benchtop centrifuge at room temperature at two thousand rpm for ten minutes to separate the serum from the blood samples. The serum of each sample was then removed by pipetting the serum into secondary tubes. The secondary tubes were pre-chilled on ice to ensure the integrity of each serum specimen by limiting degradation due to proteolysis and denaturation. The serum specimens from each sample collected were then divided into 1.0 ml aliquots in pre-chilled Cryovial tubes on ice. The aliquots from the serum specimens were stored at a temperature at least as cold as eighty degrees below Celsius (-80°C). The processing time was no more than one hour from phlebotomy to storing at -80°C.

Eight to ten serum specimens from each of the asthma population, normal population and lung cancer population were selected at random to be tested. Each serum specimen from each population was subjected to a protease or digesting agent, in this case, trypsin. Trypsin was used as the protease, and is desirable to be used as a protease because of its ability to make highly specific and highly predictable cleavages due to the fact that trypsin is known to cleave peptide chains at the carboxyl side of the lysine and arginine, except where a proline is present immediately following either the lysine or arginine. Although trypsin was used, it is possible to use other proteases or digesting agents. It is desirable to use a protease, or mixture of proteases, which cleave at least as specifically as trypsin.

The tryptic peptides, which are the peptides left by the trypsin after cleavage, were then separated from the insoluble matter by subjecting the specimens to a centrifugation and a capillary liquid chromatography, with an aqueous acetonitrile gradient with 0.1% formic acid using a 0.375 X 180 mm Supelcosil ABZ+ column on an Eksigent 2D capillary HPLC to effect chromatographic resolution of the generated tryptic peptides. This separation of the peptides is necessary because the electrospray ionization process is subject to ion co-suppression, wherein ions of a type having a higher proton affinity will suppress ion formation of ions having lower proton affinities if they are simultaneously eluting from the electrospray emitter, which in this case is co-terminal with the end of the HPLC column.

This methodology allows for the separation of the large number of peptides produced in the tryptic digestions and helps to minimize co-suppression problems, thereby maximizing chances of the formation of pseudo-molecular ion co-suppression, thereby maximizing ion sampling. The tryptic peptides for each specimen were then subjected to an LC-ESIMS. The LC-ESIMS separated each peptide in each specimen in time by passing the peptides in each specimen through a column of solvent system consisting of water, acetonitrile and formic acid as described above.

The peptides were then sprayed with in an electrospray ionization source to ionize the peptides and produce the peptide pseudo-molecular ions as described above. The peptides were passed through a mass analyzer in the LC-ESIMS where molecular masses were measured for each peptide pseudo-molecular ion. After passing through the LC-ESIMS, mass spectral readouts were produced for the peptides present in each sample from the mass spectral data, namely the intensities the molecular weights and the time of elution from a chromatographic column of the peptides. The mass spectral readouts are generally graphic illustrations of the peptide pseudo-molecular ion signals recorded by the LC-ESIMS, wherein the x-axis is the measurement of mass to charge ratio, the y-axis is the intensity of the pseudo-molecular ion signal. These data are then processed by a software system that controls the LC-ESIMS and acquires and stores the resultant data.

Once the mass spectral data was obtained and placed on the mass spectral readouts, a comparative analysis was performed wherein the mass spectral readouts of each serum specimen tested in the LC-ESIMS for each population was performed, both interpathologically and intrapathalogically. The mass spectral peaks were compared between each specimen tested in the normal population. The mass spectral peaks were then compared between each specimen tested in the asthma population and the lung cancer population. Once the intrapathological comparisons were performed, interpathological comparisons were performed wherein the mass spectral readouts for each specimen tested in the LC-ESIMS for the asthma population was compared against each specimen tested in the normal population. Likewise, the mass spectral readouts for each specimen tested in the LC-ESIMS for the lung cancer population was compared against each specimen tested in the normal population.

Peptides with mass spectral readouts that indicated the peptide intensities were inconsistently differentially expressed intrapathologically or were not substantially altered (less than a 10 fold variance in intensity) when comparing the asthma population or lung cancer population to the normal population were determined to be insignificant and excluded. Generally, the exclusion criteria used involved comparing the peptide peak intensities for at least half of the identified characteristic peptides for a given protein across at least ten data sets derived from the analysis of individual patient sera from each pathology. If the intensity of the majority of peptide peaks derived from given protein were at least 10 fold higher in intensity for 80% of the serum data sets, the protein was classed as differentially regulated between the two pathologic classes.

However, the identity of the proteins giving rise to the peptides that were observed to be differentially regulated were unknown and needed to be identified. To make the identification of the proteins, peptide pseudo-molecular ion signal intensities were compared across known databases which contain libraries of known proteins and peptides and suspected proteins and peptides.

The mass spectral readouts of the tryptic digests for each specimen from each of the normal, lung cancer and asthma population were inputted into a known search engine called Mascot. Mascot is a search engine known in the art which uses mass spectrometry data to identify proteins from four major sequencing databases, namely the MSDB, NCBInr, SwissProt and dbEST databases. These databases contain information on all proteins of known sequence and all putative proteins based on observation of characteristic protein transcription initiation regions derived from gene sequences. These databases are continually checked for accuracy and redundancy and are subject to continuous addition as new protein and gene sequences are identified and published in the scientific and patent literature.

As a result of the comparative analysis, eleven proteins were determined to be consistently differentially expressed between the asthma population, lung cancer population and normal population. Search criteria and parameters were inputted into the Mascot program and the mass spectral data from the mass spectral readouts for each population were run through the Mascot program. The mass spectral data entered into the Mascot program were for the all specimens of each pathology. The Mascot program then ran the mass spectral data for the peptides inputted against the sequencing databases, comparing the peak intensities and masses of each peptide to the masses and peak intensities of known peptides and proteins. Mascot then produced a search result which returned a candidate list of possible protein identification matches, commonly known as "significant matches" for each sample that was analyzed.

Significant matches are determined by the Mascot program by assigning a score called a "Mowse score" for each specimen tested. The Mowse score is an algorithm wherein the score is -10*LOG₁₀(P), where P is the probability that the observed match is a random event, which correlates into a significance p value where p is less than 0.05, which is the generally accepted standard in the scientific community. Mowse scores of approximately 55 to approximately 66 or greater are generally considered significant. The significance level varies somewhat due to specific search considerations and database parameters. The significant matches were returned for each peptide run, resulting in a candidate list of proteins.

Next, comparative analysis was performed comparing the mass spectral readouts for each specimen tested from the asthma population and the lung cancer population to each specimen tested from the normal population. Each tryptic peptide pseudo-molecular ion signal (peak) associated with an putatively identified protein that was detected in the LC-ESIMS was compared across asthma, lung cancer and normal pathologies. Peptides with mass spectral peak intensities that indicated the peptide quantities were not substantially altered when comparing the asthma population or lung cancer population to the normal population were determined to be insignificant and excluded. Generally, the exclusion criteria used involved comparing the peptide peak intensities for at least half of the identified characteristic peptides for a given protein across at least ten data sets derived from the analysis of individual patient sera from each pathology. If the intensity of the majority of peptide peaks derived from given protein were at least 10 fold higher in intensity for 80% of the serum data sets, the protein was classed as differentially regulated between the two pathologic classes.

The data from the mass spectral readouts were cross checked with the significant matches to confirm the raw data, peak identities, charge multiplicities, isotope distribution and flanking charge states. A reverse search was then performed to add peptides to the candidate list which may have been missed by the automated search through the Mascot program. The additional peptides were identified by selecting the "best match" meaning the single protein which substantially matched each parameter of the peptide compared, performing an *in silico* digest wherein the tryptic peptides and their respective molecular masses are calculated based on the known amino acid or gene sequence of the protein. These predicted peptide masses are then searched against the raw mass spectral data and any peaks identified are examined and qualified as described above. Then, all of the peptides including those automatically identified by Mascot and those identified by manual examination are entered into the mass list used by Mascot. The refined match is then used to derive the refined Mowse score, as presented below.

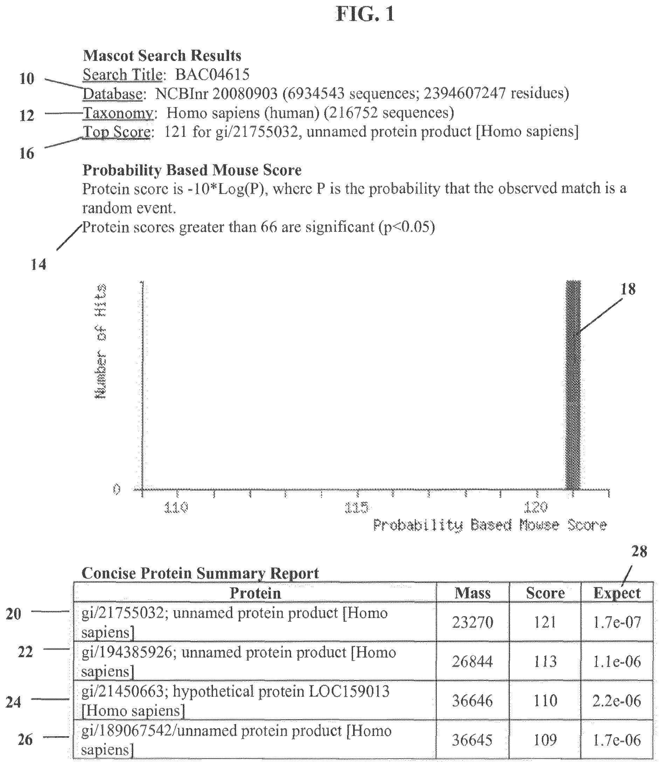

Referring to Fig. 1 through Fig. 10, Mascot search results are shown for each protein identified as differentially expressed between either the lung cancer population or the asthma population compared to the normal population. In each case, the search criteria and parameters were entered, and a Mowse score threshold for acceptability of significance was established. Referring to Fig. 1, a Mascot search result for the protein BAC04615 is shown. The database selected to be searched was NCBInr 10, and the taxonomy of the specimens entered into the Mascot program was set as Homo sapiens 12. The Mowse score threshold of significance was established as the Mowse value of sixty six or greater 14. As a result of the Mascot search, a top score of 121 was obtained, as indicated by Mowse score graph 18 the y-axis of the graph indicates the number of proteins identified having a particular Mowse score.

Still referring to Fig. 1, the top Mowse score of one hundred twenty one was given for gi/21755032, as indicated by row 20. A Mowse score of 121 is highly significant, meaning that there is a very low probability that the match is random. In fact, as indicated in column 28, the expectation that this match would occur at random is indicated by the Mascot program as 1.7 X10⁻⁰⁷. However, the proteins indicated in rows 22, 24 and 26 also had very high Mowse scores, indicating that these three proteins are significant matches as well. The manual analysis was then performed, wherein insignificant and/or noise data was removed, and raw data, peak identities, charge multiplicities, isotope distribution and flanking charge states were cross checked. As a result of the manual analysis, the probability that the proteins indicated in rows 22, 24 and 26 are significant matches was significantly reduced, and thus, proteins indicated in rows 22, 24 and 26 were excluded as matches. The protein indicated in row 20, gi/21755032, was identified as the protein indicated by the mass spectral data entered into the Mascot program in Fig. 1. The protein number indicated in row 20, gi/21755032, where gi number (sometimes written as "GI") is simply a series of digits that are assigned consecutively to each sequence record processed by NCBI. gi/21755032 corresponds to the protein BAC04615.

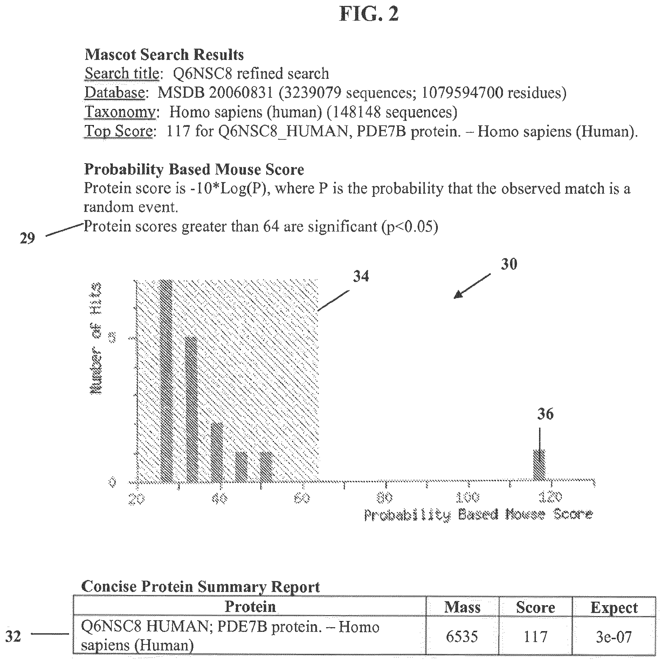

Referring to Fig. 2, a Mascot search result for the protein Q6NSC8 is disclosed. The Mowse score threshold of significance 29 was established as the Mowse value of sixty four, and a top Mowse score of one hundred seventeen was obtained, as indicated by Mowse score bar 36 in Mowse score graph 30. The protein identified which correlated to Mowse score bar 36 is Q6NSC8, as indicated in row 32. As shown in Fig 2, the shaded portion 34 of the Mowse score graph 30 indicates proteins which were recorded, but which were below the threshold of significance, and thus, were eliminated from consideration.

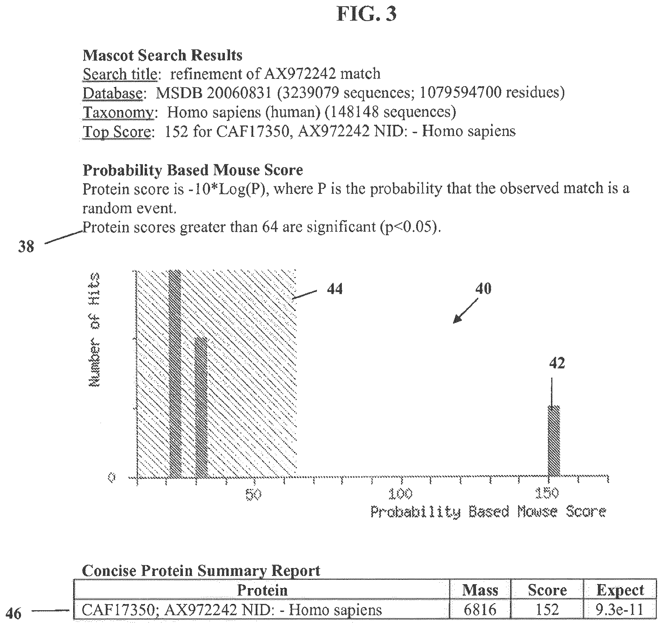

Referring to Fig. 3, a Mascot search result for the protein CAF17350 is disclosed. The Mowse score threshold of significance 38 was established as the Mowse value of sixty four, and a top Mowse score of one hundred fifty two was obtained, as indicated by Mowse score bar 42 in Mowse score graph 40. The protein identified which correlated to Mowse score bar 42 is CAF17350, as indicated in row 46. As shown in Fig 3, the shaded portion 44 of the Mowse score graph 40 indicates proteins which were recorded, but which were below the threshold of significance, and thus, were eliminated from consideration.

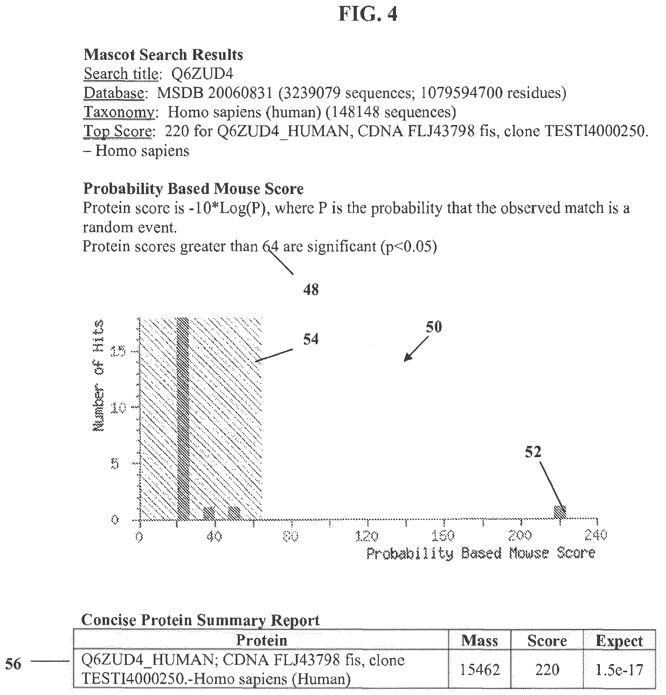

Referring to Fig. 4, a Mascot search result for the protein Q6ZUD4 is disclosed. The Mowse score threshold of significance 48 was established as the Mowse value of sixty four, and a top Mowse score of two hundred twenty was obtained, as indicated by Mowse score bar 52 in Mowse score graph 50. The protein identified which correlated to Mowse score bar 52 is Q6ZUD4, as indicated in row 56. As shown in Fig 4, the shaded portion 54 of the Mowse score graph 50 indicates proteins which were recorded, but which were below the threshold of significance, and thus, were eliminated from consideration.

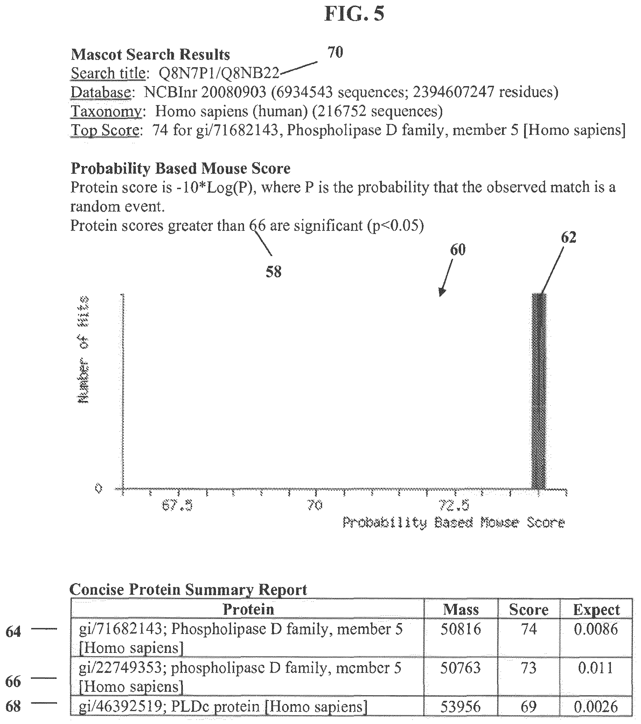

Referring to Fig. 5, a Mascot search result for the protein Q8N7P1 is disclosed. The Mowse score threshold of significance 58 was established as the Mowse value of sixty six, and a top Mowse score of seventy four was obtained, as indicated by Mowse score bar 62 in Mowse score graph 60. The protein identified which correlated to Mowse score bar 62 is gi/71682143, as indicated in row 64. Similarly to Fig. 1, gi/71682143 corresponds to protein Q8N7PI. The proteins indicated in rows 66 and 68 also had very high Mowse scores, indicating that these two proteins are significant matches as well. The manual analysis was then performed, wherein insignificant and/or noise data was removed, and raw data, peak identities, charge multiplicities, isotope distribution and flanking charge states were cross checked. As a result of the manual analysis, the probability that the proteins indicated in rows 66 and 68 are significant matches was significantly reduced, and thus, proteins indicated in rows 66 and 68 were excluded as matches. Q8N7PI was identified as the protein indicated by the mass spectral data entered into the Mascot program in Fig. 5. The indication at 70 to the protein Q8NB22 is indicated because it is the same protein as Q8N7PI.

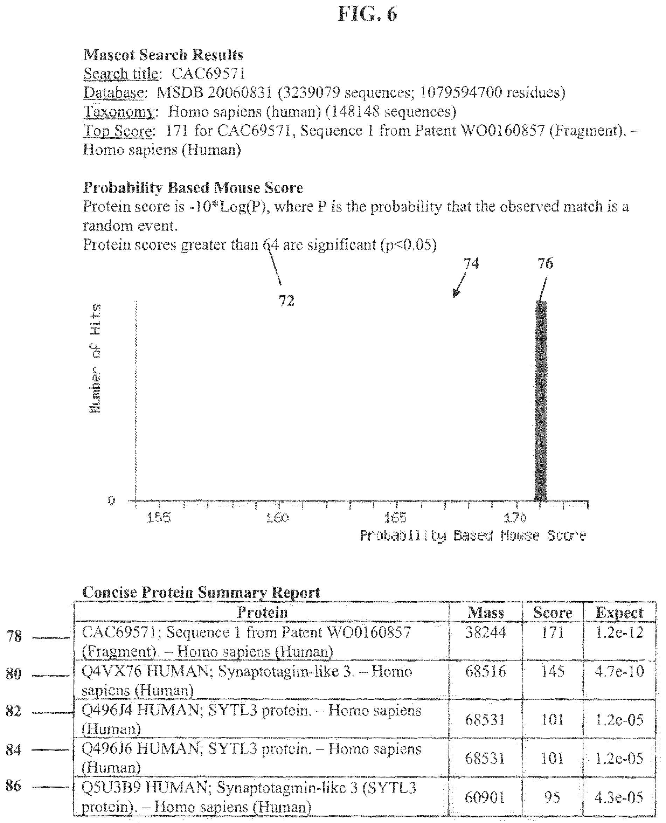

Referring to Fig. 6, a Mascot search result for the protein CAC69571 is disclosed. The Mowse score threshold of significance 72 was established as the Mowse value of sixty four, and a top Mowse score of one hundred seventy one was obtained, as indicated by Mowse score bar 76 in Mowse score graph 74. The protein indicated which correlated to Mowse score bar 76 is CAC69571, as indicated in row 78. The proteins indicated in rows 80, 82, 84 and 86 also had very high Mowse scores, indicating that these four proteins are significant matches as well. The manual analysis was then performed, wherein insignificant and/or noise data was removed, and raw data, peak identities, charge multiplicities, isotope distribution and flanking charge states were cross checked. As a result of the manual analysis, the probability that the proteins indicated in rows 80, 82, 84 and 86 are significant matches was significantly reduced, and thus, proteins indicated in rows 80, 82, 84 and 86 were excluded as matches. CAC69571 was identified as the protein indicated by the mass spectral data entered into the Mascot program in Fig. 6.

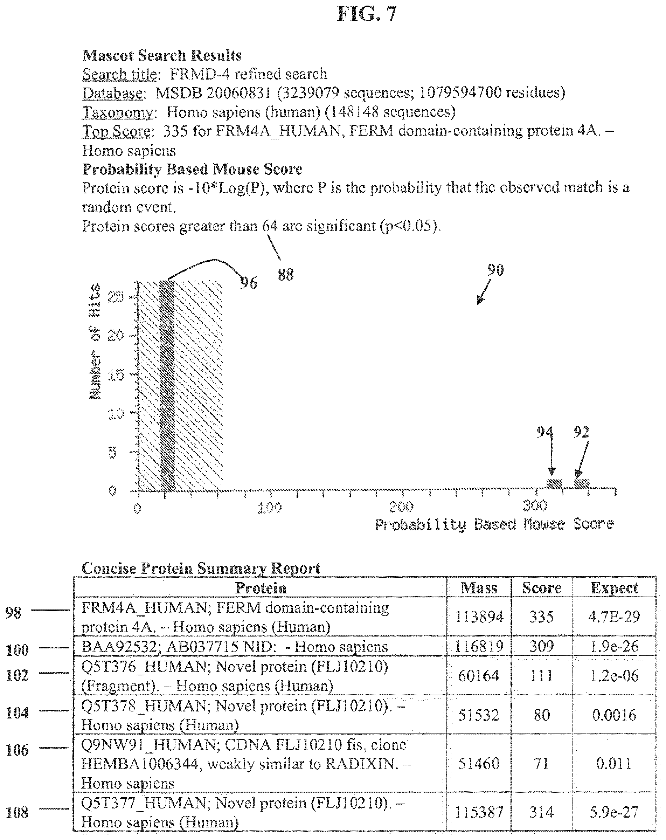

Referring to Fig. 7, a Mascot search result for the protein FERM 4 domain containing protein 4 is disclosed. The Mowse score threshold of significance 88 was established as the Mowse value of sixty four, and a top Mowse score of three hundred thirty five was obtained, as indicated by Mowse score bar 92 in Mowse score graph 90. The protein indicated which correlated to Mowse score bar 92 is FERM 4 domain containing protein 4, as indicated in row 98. The proteins indicated in rows 100, 102, 104 and 106 and 108 also had very high Mowse scores, indicating that these five proteins are significant matches as well. The manual analysis was then performed, wherein insignificant and/or noise data was removed, and raw data, peak identities, charge multiplicities, isotope distribution and flanking charge states were cross checked. As a result of the manual analysis, the probability that the proteins indicated in rows 100, 102, 104 and 106 and 108 are significant matches was significantly reduced, and thus, proteins indicated in rows 100, 102, 104 and 106 and 108 were excluded as matches. FERM 4 domain containing protein 4 was identified as the protein indicated by the mass spectral data entered into the Mascot program in Fig. 7.

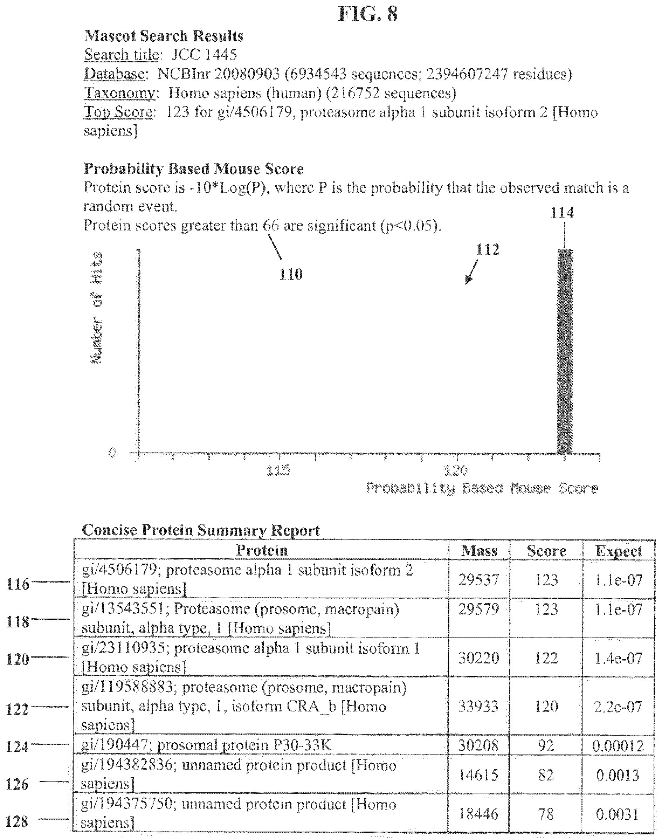

Referring to Fig. 8, a Mascot search result for the protein JCC1445 proteasome endopeptidase complex chain C2 long splice form ("JCC 1445") is disclosed. The Mowse score threshold of significance 110 was established as the Mowse value of sixty six, and a top Mowse score of one hundred twenty three was obtained, as indicated by Mowse score bar 114 in Mowse score graph 112. The protein identified which correlated to Mowse score bar 114 is gi/4506179, as indicated in row 116. gi/4506179 corresponds to protein JCC1445. The proteins indicated in rows 118, 120, 122, 124, 126 and 128 also had very high Mowse scores, indicating that these six proteins are significant matches as well. The manual analysis was then performed, wherein insignificant and/or noise data was removed, and raw data, peak identities, charge multiplicities, isotope distribution and flanking charge states were cross checked. As a result of the manual analysis, the probability that the proteins indicated in rows 118, 120, 122, 124, 126 and 128 are significant matches was significantly reduced, and thus, proteins indicated in rows 118, 120, 122, 124, 126 and 128 were excluded as matches. JCC1445 was identified as the protein indicated by the mass spectral data entered into the Mascot program in Fig. 8.

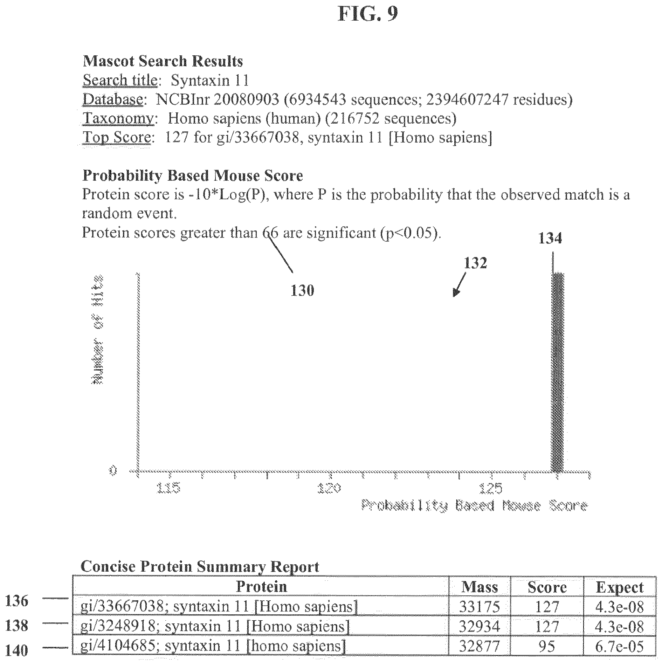

Referring to Fig. 9, a Mascot search result for the protein Syntaxin 11 is disclosed. The Mowse score threshold of significance 130 was established as the Mowse value of sixty six, and a top Mowsc score of one hundred twenty seven was obtained twice, as indicated by Mowse score bars 134, and rows 136 and 138. A third Mowse score of 95 was obtained for Syntaxin 11, as indicated in row 140. Syntaxin 11 was identified as the protein indicated by the mass spectral data entered into the Mascot program in Fig. 9.

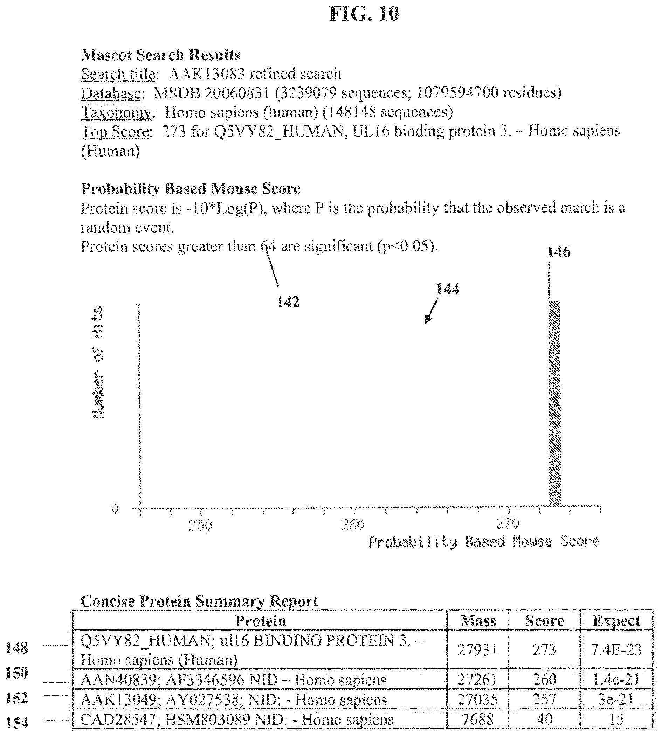

Referring to Fig. 10, Mascot search results for two proteins, AAK13083 and AAK13049 are disclosed. The Mowse score threshold of significance 142 was established as the Mowse value of sixty four, and a top Mowse score of two hundred seventy three was obtained by protein Q5VY82, as indicated in row 148 and Mowse score bar 146. The proteins indicated in rows 150, 152 and 154 also had very high Mowse scores, indicating that these three proteins are significant matches as well. However, as a result of the manual analysis performed, the proteins indicated in rows 150 and 154 were eliminated as probable matches. Q5VY82 is undergoing further investigation and experimentation to determine whether it is significantly differentially expressed. AAK13049, as indicated in row 152 and AAK13083 were both identified as proteins indicated by the mass spectral data entered into the Mascot program in Fig. 10.

Fig. 1 through Fig. 10 disclose data analysis that was performed to identify the eleven proteins which are differentially expressed in asthma and/or lung cancer populations when compared to the normal populations. The process described herein, and as indicated in Fig. 1 through Fig. 10 was performed for each of the eleven proteins, for the asthma population, normal population and lung cancer population.

As a result of the identification process, the eleven proteins determined to be significantly differentially expressed between the asthma population, lung cancer population and/or normal population were identified as BAC04615, Q6NSC8, CAF17350, Q6ZUD4, Q8N7P1, CAC69571, FERM domain containing protein 4, JCC1445 proteasome endopeptidase complex chain C2 long splice form, Syntaxin 11, AAK13083, and AAK130490. BAC04615, Q6NSC8, CAF 17350, Q6ZUD4, Q8N7P1 are identified proteins resulting from genetic sequencing efforts. FERM domain containing protein 4 is known to be involved in intracytoplasmic protein membrane anchorage. JCC1445 proteasome endopeptidase complex chain C2 long splice form is a known proteasome. Syntaxin 11 is active in cellular immune response. BAC04615, AAK13083, and AAK130490 are major histocompatibility complex ("MHC") associated proteins.

Having identified eleven specific proteins which are consistently differentially expressed in asthma and lung cancer patients, it is possible to diagnose these pathologies early in the progression of the diseases by subjecting the proteins BAC04615, Q6NSC8, CAF17350, Q6ZUD4, Q8N7P1, CAC69571, FERM domain containing protein 4, JCC1445 proteasome endopeptidase complex chain C2 long splice form, Syntaxin 11, AAK13083, and AAK130490 from a patient's serum to the LC-ESIMS, obtaining the mass spectral data, from these proteins, and comparing the mass spectral data to mass spectral data of normal populations. Further analysis can be performed by comparing the mass spectral data to mass spectral data from lung cancer populations and/or asthma populations to verify or nullify the presence of the given pathologies.

The analysis could, of course, be extended to multiple additional techniques whereby specific protein concentrations can be determined, including but not limited to: Radio-immuno Assay, enzyme linked immuno sorbent assay, high pressure liquid chromatography with radiometric, spectrometric detection via absorbance of visible or ultraviolet light, mass spectrometric qualitiative and quantitative analysis, western blotting, 1 or 2 dimensional gel electrophoresis with quantitative visualization by means of detection of radioactive probes or nuclei, antibody based detection with absorptive or fluorescent photometry, quantitation by luminescence of any of a number of chemiluminescent reporter systems, enzymatic assays, immunoprecipitation or immuno-capture assays, or any of a number of solid and liquid phase immuno assays.

In addition to determining the existence of lung cancer or asthma early in the development of the disease, the proteins identified herein as indicative of such pathologies could be used and applied in related ways to further the goal of treating lung cancer and/or asthma. For instance, antibodies can be developed to bind to these proteins. The antibodies could be assembled in a biomarker panel wherein any or all of the antibodies are assembled into a single bead based panel or kit for a bead based immunoassay. The proteins could then be subjected to a multiplexed immunoassay using bead based technologies, such as Luminex's xMAP technologies, and quantified. Furthermore, other non-bead based assays could be used to quantify the protein expression levels. By quantifying the protein expression levels, those quantifiable results can be compared to expression levels of normal populations, asthma populations, and/or lung cancer populations to further verify or nullify the presence of lung cancer or asthma in the patient.

The proteins could also be used and applied to the field of pharmacology to evaluate the response of a patient to therapeutic interventions such as drug treatment, radiation/chemotherapy, or surgical treatment. Furthermore, kits to measure individual proteins or a panel of the proteins could be used for routine testing of a patient to monitor health status of a patient who is at greater risk of the pathologies, such as smokers, or those with family histories of the pathologies.

Finally, a Sequence Listing of the amino acid sequences for each of the eleven proteins identified herein is provided herewith. In the Sequence Listing, the amino acid sequence disclosed in SEQ ID NO: 1 is the primary amino acid sequence known as of the date of filing this application for the protein BAC04615. The amino acid sequence disclosed in SEQ ID NO: 2 is the primary amino acid sequence known as of the date of filing this application for the protein Q6NSC8. The amino acid sequence disclosed in SEQ ID NO: 3 is the primary amino acid sequence known as of the date of filing this application for the protein CAF17350. The amino acid sequence disclosed in SEQ ID NO: 4 is the primary amino acid sequence known as of the date of filing this application for the protein Q6ZUD4. The amino acid sequence disclosed in SEQ ID NO: 5 is the primary amino acid sequence known as of the date of filing this application for the protein FERM domain containing protein 4. The amino acid sequence disclosed in SEQ ID NO: 6 is the primary amino acid sequence known as of the date of filing this application for the protein AAK13083. The amino acid sequence disclosed in SEQ ID NO: 7 is the primary amino acid sequence known as of the date of filing this application for the protein Q8N7P1. The amino acid sequence disclosed in SEQ ID NO: 8 is the primary amino acid sequence known as of the date of filing this application for the protein CAC69571. The amino acid sequence disclosed in SEQ ID NO: 9 is the primary amino acid sequence known as of the date of filing this application for the protein JCC1445 proteasome endopetidase complex chain C2 long splice. The amino acid sequence disclosed in SEQ ID NO: 10 is the primary amino acid sequence known as of the date of filing this application for the protein Syntaxin 11. The amino acid sequence disclosed in SEQ ID NO: 11 is the primary amino acid sequence known as of the date of filing this application for the protein AAK13049.
## Claims

1. A method aiding in the diagnosis of asthma and/or lung cancer pathologies in patients early in the progression of the diseases comprising determining the specific protein concentrations of CAC69571 (SEQ ID NO: 8), and optionally one or more of the proteins FERM domain containing proteins 4 (SEQ ID NO: 5), JC1445 proteasome endopetidase complex chain C2 long splice (SEQ ID NO: 9), Syntaxin 11 (SEQ ID NO: 10, AAK13083 (SEQ ID NO: 6), AAK13049 (SEQ ID NO: 11), BACO4615 (SEQ ID NO: 1), Q6NSC8 (SEQ ID NO: 2), CAF17350 (SEQ ID NO: 3), Q6ZUD4 (SEQ ID NO: 4), and/or Q8N7P1 (SEQ ID NO: 7) in a patient's serum and comparing the concentration data to concentration data from lung cancer populations and/or asthma populations to verify or nullify the presence of the given pathologies.

2. The method of claim 1, wherein specific protein concentrations are determined by obtaining the mass spectral data, Radio-immuno Assay, enzyme linked immuno sorbent assay, high pressure liquid chromatography with radiometric, spectrometric detection via absorbance of visible or ultraviolet light, mass spectrometric qualitative and quantitative analysis, western blotting, 1 or 2 dimensional gel electrophoresis with quantitative visualization by means of detection of radioactive probes or nuclei, antibody based detection with absorptive or fluorescent photometry, quantitation by luminescence of any of a number of chemiluminescent reporter systems, enzymatic assays, immunoprecipitation or immuno-capture assays, or any of a number of solid and liquid phase immuno assays.

3. A method to evaluate the response of a patient to therapeutic interventions comprising determining the specific protein concentrations of CAC69571 (SEQ ID NO: 8), and optionally one or more of the proteins FERM domain containing proteins 4 (SEQ ID NO: 5), JC1445 proteasome endopetidase complex chain C2 long splice (SEQ ID NO: 9), Syntaxin 11 (SEQ ID NO: 10), AAK13083 (SEQ ID NO: 6), AAK13049 (SEQ ID NO: 11), BACO4615 (SEQ ID NO: 1), Q6NSC8 (SEQ ID NO: 2), CAF17350 (SEQ ID NO: 3), Q6ZUD4 (SEQ ID NO: 4), and/or Q8N7P1 (SEQ ID NO: 7) in a patient's serum, wherein the patient is an asthma or lung cancer patient.

4. The method of claim 3, wherein the therapeutic intervention is drug treatment, radiation/chemotherapy, or surgical treatment.

5. The method of any preceding claim, comprising determining the specific protein concentrations of CAC69571 (SEQ ID NO: 8), and at least one of the proteins FERM domain containing proteins 4 (SEQ ID NO: 5), JC1445 proteasome endopetidase complex chain C2 long splice (SEQ ID NO: 9), Syntaxin 11 (SEQ ID NO: 10), AAK13083 (SEQ ID NO: 6), AAK13049 (SEQ ID NO: 11), BACO4615 (SEQ ID NO: 1), Q6NSC8 (SEQ ID NO: 2), CAF17350 (SEQ ID NO: 3), Q6ZUD4 (SEQ ID NO: 4), and/or Q8N7P1 (SEQ ID NO: 7) in a patient's serum, wherein the patient is an asthma or lung cancer patient.

## Patentansprüche

1. Verfahren zur Unterstützung der Diagnose von Asthma- und/oder Lungenkrebspathologien bei Patienten im frühen Verlauf der Erkrankungen, umfassend die Bestimmung der spezifischen Proteinkonzentrationen von CAC69571 (SEQ ID NR.: 8) und wahlweise von einem oder mehreren der Proteine FERM-Domäne-haltiges Protein 4 (SEQ ID NR.: 5), JC1445 Proteasom-Endopeptidase-Komplex, Kette C2, lange Spleißform (SEQ ID NR.: 9), Syntaxin 11 (SEQ ID NR.: 10), AAK13083 (SEQ ID NR.: 6), AAK13049 (SEQ ID NR.: 11), BACO4615 (SEQ ID NR.: 1), Q6NSC8 (SEQ ID NR.: 2), CAF17350 (SEQ ID NR.: 3), Q6ZUD4 (SEQ ID NR.: 4) und/oder Q8N7P1 (SEQ ID NR.: 7) im Serum eines Patienten und Vergleich der Konzentrationsdaten mit Konzentrationsdaten aus Lungenkrebs-Populationen und/oder Asthma-Populationen, um die Präsenz der gegebenen Pathologien zu verifizieren oder zu nullifizieren.

2. Verfahren nach Anspruch 1, wobei spezifische Proteinkonzentrationen bestimmt werden durch Ermittlung der Massenspektrendaten, Radioimmunassay, Enzyme Linked Immunosorbent Assay, Hochdruck-Flüssigkeitschromatographie mit radiometrischer, spektrometrischer Detektion durch Absorbanz von sichtbarem oder ultraviolettem Licht, massenspektrometrische qualitative und quantitative Analyse, Western Blotting, 1- oder 2-dimensionale Gelelektrophorese mit quantitativer Visualisierung mittels Detektion von radioaktiven Sonden oder Kernen, antikörperbasierte Detektion mit Absorptions- oder Fluoreszenzphotometrie, Quantifizierung durch Lumineszenz beliebiger einer Reihe von chemilumineszenten Reportersystemen, enzymatischen Assays, Immunpräzipitation oder Immuno-Capture-Assays oder beliebige einer Reihe von Fest- und Flüssigphasen-Immunassays.

3. Verfahren zur Evaluierung des Ansprechens eines Patienten auf therapeutische Interventionen, umfassend die Bestimmung der spezifischen Proteinkonzentrationen von CAC69571 (SEQ ID NR.: 8) und wahlweise von einem oder mehreren der Proteine FERM-Domäne-haltiges Protein 4 (SEQ ID NR.: 5), JC1445 Proteasom-Endopeptidase-Komplex, Kette C2, lange Spleißform (SEQ ID NR.: 9), Syntaxin 11 (SEQ ID NR.: 10), AAK13083 (SEQ ID NR.: 6), AAK13049 (SEQ ID NR.: 11), BACO4615 (SEQ ID NR.: 1), Q6NSC8 (SEQ ID NR.: 2), CAF17350 (SEQ ID NR.: 3), Q6ZUD4 (SEQ ID NR.: 4) und/oder Q8N7P1 (SEQ ID NR.: 7) im Serum eines Patienten, wobei der Patient ein Asthma- oder Lungenkrebspatient ist.

4. Verfahren nach Anspruch 3, wobei die therapeutische Intervention in medikamentöser Behandlung, Strahlen-/Chemotherapie oder operativer Behandlung besteht.

5. Verfahren nach einem vorhergehenden Anspruch, umfassend die Bestimmung der spezifischen Proteinkonzentrationen von CAC69571 (SEQ ID NR.: 8) und von mindestens einem der Proteine FERM-Domäne-haltiges Protein 4 (SEQ ID NR.: 5), JC1445 Proteasom-Endopeptidase-Komplex, Kette C2, lange Spleißform (SEQ ID NR.: 9), Syntaxin 11 (SEQ ID NR.: 10), AAK13083 (SEQ ID NR.: 6), AAK13049 (SEQ ID NR.: 11), BACO4615 (SEQ ID NR.: 1), Q6NSC8 (SEQ ID NR.: 2), CAF17350 (SEQ ID NR.: 3), Q6ZUD4 (SEQ ID NR.: 4) und/oder Q8N7P1 (SEQ ID NR.: 7) im Serum eines Patienten, wobei der Patient ein Asthma- oder Lungenkrebspatient ist.

## Revendications

1. Procédé facilitant le diagnostic de l'asthme et/ou de pathologies de cancer du poumon chez des patients à un stade précoce dans la progression des maladies, consistant à déterminer les concentrations de protéines spécifiques de CAC69571, (SEQ ID NO 8), et éventuellement d'une ou plusieurs protéines parmi les protéines 4 contenant un domaine FERM (SEQ ID NO 5), la forme longue épissée de C2 à chaîne de complexe protéasome-endopeptidase JC1445 (SEQ ID NO 9), la syntaxine 11 (SEQ ID NO 10), AAK13083 (SEQ ID NO 6), AAK13049 (SEQ ID NO 11), BACO4615 (SEQ ID NO 1), Q6NSC8 (SEQ ID NO 2), CAF17350 (SEQ ID NO 3), Q6ZUD4 (SEQ ID NO 4) et/ou Q8N7P1 (SEQ ID NO 7) dans le sérum d'un patient, et comparer les données de concentration à des données de concentration de populations atteintes d'un cancer du poumon et/ou de populations asthmatiques afin de confirmer ou d'infirmer la présence des pathologies données.

2. Procédé selon la revendication 1, dans lequel les concentrations de protéines données sont déterminées au moyen de données spectrales massiques, d'un test radio-immunologique, d'un titrage avec un immunoabsorbant lié à une enzyme, d'une chromatographie en phase liquide à haute pression avec détection radiométrique ou spectrométrique via absorption de la lumière visible ou ultraviolette, d'une analyse qualitative et quantitative par spectrométrie de masse, d'une technique de Western blot, d'une électrophorèse sur gel unidimensionnelle ou bidimensionnelle avec visualisation quantitative par détection de sondes radioactives ou détection de noyaux ou d'anticorps avec photométrie d'absorption ou de fluorescence, d'une quantification par luminescence d'un système reporter chimioluminescent parmi un certain nombre de systèmes reporters chimioluminescents, d'essais enzymatiques, de dosages d'immunoprécipitation et d'immunocapture, ou d'un essai immunologique en phase solide et liquide quelconque parmi un certain nombre d'essais immunologiques en phase solide et liquide.

3. Procédé d'évaluation de la réponse d'un patient à des interventions thérapeutiques, consistant à déterminer les concentrations de protéines spécifiques de CAC69571 (SEQ ID NO 8), et éventuellement d'une ou plusieurs protéines parmi les protéines 4 contenant un domaine FERM (SEQ ID NO 5), la forme longue épissée de C2 à chaîne de complexe protéasome-endopeptidase JC1445 (SEQ ID NO 9), la syntaxine 11 (SEQ ID NO 10), AAK13083 (SEQ ID NO 6), AAK13049 (SEQ ID NO 11), BACO4615 (SEQ ID NO 1), Q6NSC8 (SEQ ID NO 2), CAF17350 (SEQ ID NO 3), Q6ZUD4 (SEQ ID NO 4) et/ou Q8N7P1 (SEQ ID NO 7) dans le sérum d'un patient, le patient étant un patient asthmatique ou atteint d'un cancer du poumon.

4. Procédé selon la revendication 3, dans lequel l'intervention thérapeutique est un traitement médicamenteux, une radiothérapie/chimiothérapie, ou un traitement chirurgical.

5. Procédé selon l'une quelconque des revendications précédentes, consistant à déterminer les concentrations de protéines spécifiques de CAC69571 (SEQ ID NO 8), et au moins une des protéines parmi les protéines 4 contenant un domaine FERM (SEQ ID NO 5), la forme longue épissée de C2 à chaîne de complexe protéasome-endopeptidase JCC1445 (SEQ ID NO 9), la syntaxine 11 (SEQ ID NO 10), AAK13083 (SEQ ID NO 6), AAK13049 (SEQ ID NO 11), BACO4615 (SEQ ID NO 1), Q6NSC8 (SEQ ID NO 2), CAF17350 (SEQ ID NO 3), Q6ZUD4 (SEQ ID NO 4) et/ou Q8N7P1 (SEQ ID NO 7) dans le sérum d'un patient, le patient étant un patient asthmatique ou atteint d'un cancer du poumon.
